# FASCICULE DE BREVET EUROPEEN

(11) **EP 1 268 384 B1**
(45) Date de publication et mention de la délivrance du brevet: **19.10.2005**
(21) Numéro de dépôt: 01913955.9
(22) Date de dépôt: 07.03.2001
(51) Int. Cl.: C07C 51/215, C07C 51/31, C07C 55/14, C07C 55/21

(54) **PROCEDE D'OXYDATION DE CYCLOHEXANE EN ACIDES**
VERFAHREN ZUR OXIDATION VON CYCLOHEXAN IN CARBONSÄUREN
METHOD FOR OXIDISING CYCLOHEXANE IN ACIDS

(30) Priorité: 08.03.2000 FR 0002996
(43) Date de publication de la demande: 02.01.2003
(73) Titulaire: RHODIA POLYAMIDE INTERMEDIATES, 69192 Saint-Fons (FR)
(72) Inventeur: FACHE, Eric, F-69300 Caluire et Cuire (FR)
(74) Mandataire: Esson, Jean-Pierre
(86) Numéro de dépôt international: PCT/FR2001/000686
(87) Numéro de publication internationale: WO 2001/066502

(56) Documents cités:
- EP-A- 0 824 962
- WO-A-00/46172
- FR-A- 2 732 678
- GB-A- 628 457
- US-A- 3 907 881
- US-A- 4 032 569
- US-A- 6 147 256

## Description

La présente invention concerne un procédé d'oxydation par un agent oxydant contenant de l'oxygène moléculaire de cyclohexane en acide adipique.

L'oxydation du cyclohexane en acide adipique est un procédé qui a été étudié depuis de nombreuses années. En effet, l'acide adipique est un composé chimique important utilisé comme matière première dans de nombreuses fabrications telles que la production de polymères comme les polyamides, polyesters ou polyuréthannes.

Plusieurs procédés de fabrication d'acide adipique à partir d'hydrocarbures tels que benzène, phénol, cyclohexéne cyclohexane ont été proposés.

L'oxydation du cyclohexane soit directement soit en deux étapes sont les voies les plus avantageuses pour produire l'acide adipique.

Ainsi, le brevet américain 2,223,493 publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

De nombreux autres brevets et articles décrivent cette réaction d'oxydation directe du cyclohexane en acide adipique. Toutefois, pour obtenir des rendements acceptables de production d'acide adipique, ces documents décrivent l'utilisation de l'acide acétique comme solvant, en présence soit de catalyseur homogène soit de catalyseur hétérogène. On peut citer, à titre d'illustration, l'article paru dans le journal "Chemtech", 555-559 (septembre 1974) dont l'auteur est K. Tanaka qui résume et commente le procédé d'oxydation directe du cyclohexane. On peut également citer les brevets américains 3,231,608 ; 4,032,569 ; 4,158,738:4.263.453 et 5,321,157., le brevet européen 870751 qui décrivent différents systèmes catalytiques homogènes.

Il a également été proposé des procédés d'oxydation directe du cyclohexane en présence de catalyseur hétérogène comme des aluminophosphates substitués par du cobalt, comme dans le brevet européen n°519569.

Le choix du solvant, à savoir l'acide acétique, est une caractéristique importante pour obtenir un taux de transformation du cyclohexane et une production d'acide adipique acceptables. L'utilisation d'un tel solvant présente de nombreux inconvénients provoqués par, par exemple, son caractère corrosif aux conditions de température et de pression utilisées. De plus, l'utilisation de ce solvant pose de nombreux problèmes pour les étapes de séparation et extraction de l'acide adipique produit et le recyclage de divers composés.

En effet, en présence d'acide acétique, il est difficile de séparer et extraire du milieu réactionnel les composés sous-produits de l'oxydation tels que la cyclohexanone et le cyclohexanol formés

En outre, l'extraction de l'acide adipique par cristallisation et sa purification sont rendues difficiles car la solubilité à froid de cet acide est plus élevée à 25°C dans l'acide acétique et moins élevée à 80°C dans l'acide acétique que dans l'eau.

La séparation et le recyclage du catalyseur homogène sont également difficiles en présence d'acide acétique. En effet, d'une part un recyclage du catalyseur sans extraction de celui-ci ne permet pas de conserver une activité catalytique suffisante, et d'autre part les opérations de séparation du catalyseur avant recyclage comme décrites notamment dans les brevets français n° 2722783, 2746671, sont complexes et coûteuses.

De plus, ce solvant impose de réaliser une déshydratation difficile et coûteuse du milieu réactionnel.

Il a également été proposé quelques procédés d'oxydation en une seule étape du cyclohexane en acide adipique sans utilisation d'acide acétique. Certains proposent de réaliser cette réaction en l'absence de solvants, d'autres avec des solvants tels que des esters organiques comme les acétates (US 4,098,817), de l'acétone (US 2,589,648) ou encore des alcools comme le butanol, le méthanol, le cyclohexanol ou l'acétonitrile (EP 784045).

Ces procédés conduisent généralement à des sélectivités en acide adipique très faibles. Par ailleurs, les solvants utilisés présentent souvent une faible stabilité dans les conditions d'oxydation de l'hydrocarbure comme le cyclohexane. Cette faible stabilité provoque une consommation importante du solvant ce qui rend inexploitable de tels procédés.

Un des buts de la présente invention est de proposer un procédé d'oxydation d'hydrocarbures en une seule étape pour produire des acides ou polyacides, dans un milieu liquide aux conditions de la réaction d'oxydation et permettant une séparation de l'acide produit et un recyclage notamment du catalyseur par des opérations simples.

A cet effet, l'invention propose un procédé d'oxydation de cyclohexane en acide adipique dans un milieu liquide par un agent d'oxydation comprenant de l'oxygène moléculaire, caractérisé en ce qu'un des constituants du milieu liquide est un composé organique acide insoluble dans l'eau, ou à caractère lipophile choisi dans le groupe comprenant les acides 2,5-ditertiobutyl benzoïque, 4-tertiobutylbenzoïque, 4-octylbenzoïque, l'hydrogénoorthophtalate de tertiobutyle, les acides naphténiques ou anthracéniques substitués par des groupements -alkyles, de préférence de type tertiobutyle, les dérivés substitués des acides phtaliques.

Selon l'invention, le composé acide lipophile est un composé qui doit former aux conditions de température et de pression de la réaction d'oxydation, au moins une phase liquide homogène avec le ou les hydrocarbures à oxyder. Ainsi, le composé acide lipophile pourra avantageusement être au moins partiellement miscible avec le ou les hydrocarbures à oxyder, aux conditions de température et de pression mises en oeuvre pour réaliser la réaction d'oxydation.

Par au moins partiellement miscible, on entend qu'aux conditions de la réaction d'oxydation, la solubilité d'un composé dans l'autre soit au moins supérieure à 2% en poids, et qu'au moins une phase liquide homogène comprenant au moins une partie des hydrocarbures à oxyder et du composé acide lipophile soit formée.

Dans un mode de réalisation préféré de l'invention, la miscibilité entre l'hydrocarbure et le composé acide lipophile est telle qu'aux conditions de mise en oeuvre de l'invention, ces deux composés forment une seule phase liquide homogène:

Par composé acide lipophile convenable pour l'invention, on entend les composés présentant une faible solubilité dans l'eau, c'est à dire une solubilité inférieure à 10 % en poids à température ambiante(10°C ; 30°C).

Toutefois, il est possible sans sortir du cadre de l'invention, d'utiliser des composés organiques présentant une solubilité dans l'eau supérieure à celle indiquée précédemment si le coefficient de partage de ce composé entre la ou les phases organiques du milieu réactionnel constituées essentiellement par l'hydrocarbure à oxyder, les intermédiaires d'oxydation et la phase non organique comprenant l'eau formée pendant la réaction d'oxydation permet d'obtenir une concentration du composé organique lipophile dans ladite phase aqueuse inférieure à 10 % en poids.

Comme composé organique lipophile on peut citer par exemple, 2,5-ditertiobutyl benzoïque, 4-tertiobutylbenzoïque, 4-octylbenzoïque, l'hydrogénoorthophtalate de tertiobutyle, les acides naphténiques ou anthracéniques substitués par des groupements alkyles, de préférence de type tertiobutyle, les dérivés substitués des acides phtaliques.

Selon une autre caractéristique de l'invention, la concentration en composé acide lipophile dans le milieu réactionnel est déterminée pour obtenir un rapport molaire entre le nombre de mole d'acide lipophile et le nombre de mole de métal formant le catalyseur compris entre 0,5 et 100 000, de préférence entre 1 et 1000.

La concentration en composé acide lipophile dans le milieu liquide d'oxydation peut varier dans de larges limites. Ainsi, il peut être compris entre 1 et 99 % en poids par rapport au poids total du milieu liquide, plus avantageusement il peut être compris entre 10 et 80 % en poids du milieu liquide.

Il est également possible, sans pour cela sortir du cadre de l'invention, d'utiliser le composant acide iipophile en association avec un autre composé qui peut notamment avoir comme effet d'améliorer la productivité et/ou la sélectivité de la réaction d'oxydation en acide adipique, et notamment la solubilisation de l'oxygène.

Comme exemple de tels composés, on peut citer, en particulier, les nitriles, les composés hydroxyimides les composés halogénés, plus avantageusement les composés fluorés. Comme composés plus particulièrement convenables, on peut citer les nitriles comme l'acétonitrile, le benzonitrile, les imides appartenant à la famille décrite dans la demande de brevet EP 0824962, et plus particulièrement la N-hydroxysuccinimide (NHS) ou la N-hydroxyphtalimide (NHP1), des dérivés halogénés comme le dichlorométhane, les composés fluorés comme :
- Hydrocarbures aliphatiques fluorés ou perfluorés cycliques ou acycliques, hydrocarbures fluorés aromatiques tels le perfluorotoluène, perfluorométhylcyclohexane, perfluorohexane, perfluoroheptane, perfluorooctane, perfluorononane, perfluorodécaline, perfluorométhyldécaline, α,α,α-trifluorotofuène, 1,3-bis(méthyl trifluoro)benzène.
- Esters perfluorés ou fluorés tels que perfluorooctanoates d'alkyle, perfluoronanoates d'alkyle
- Cétones fluorés ou perfluorés telles que acétone perfluorée
- Alcools fluorés ou perfluorés tels que hexanol, octanol, nonanol, décanol perfluorés, t-butanol perfluoré, isopropanol perfluoré, hexafluoro-1,1,1,3,3.3-propanol-2
- Nitriles fluorés ou perfluorés tels que acétonitrile perfluoré
- Acides fluorés ou perfluorés tels que acides trifluorométhyl benzoïque, acide pentafluorobenzoique, acide hexanoique, heptanoique, octanoique, nonanoique perfluorés, acide adipique perfluoré
- Halogénures fluorés ou perfluorés tels que iodo octane perfluoré, bromooctane perfluoré
- Amines fluorées ou perfluorées tels que tripropylamine perfluorée, tributylamine perfluorée, tripentylamine perfluorée :

L'oxydation est réalisée, en général, en présence d'un catalyseur. Ce catalyseur comprend avantageusement un élément métallique choisi dans le groupe comprenant Cu, Ag, Au, Mg, Ca, Sr, Ba, Zn, Cd, Hg, Al, Sc, In, Tl, Y, Ga, Ti, Zr, Hf, Ge, Sn, Pb, V, Nb, Ta, Cr, Mo, W, Mn, Tc, Re, Fe, Ru, Os, Co, Rh, Ir, Ni, Pd, Pt, les lanthanides comme Ce et les combinaisons de ceux-ci.

Ces éléments catalytiques sont mis en oeuvre soit sous forme de composés avantageusement au moins partiellement solubles dans le milieu liquide d'oxydation aux conditions de mise en oeuvre de la réaction d'oxydation (catalyse homogène), soit supportés, absorbés ou liés à un support inerte tel que silice, alumine, par exemple (catalyse hétérogène).

Le catalyseur est de préférence, notamment aux conditions de mise en oeuvre de la réaction d'oxydation :
- Soit soluble dans l'hydrocarbure à oxyder,
- Soit soluble dans le composé acide lipophile,
- Soit soluble dans le mélange hydrocarbure/composé acide lipophile formant une phase liquide homogène aux conditions de mise en oeuvre de la réaction.

Selon un mode de réalisation préféré de l'invention, le catalyseur utilisé est soluble dans l'un de ces milieux à température ambiante ou à la température de recyclage de ces milieux dans une nouvelle oxydation.

Par le terme soluble, on entend que le catalyseur soit au moins partiellement soluble dans le milieu considéré.

Dans le cas d'une catalyse hétérogène, les éléments métalliques catalytiquement actifs sont supportés ou incorporés dans une matrice minérale micro ou mésoporeuse ou dans une matrice polymérique ou sont sous forme de complexes organométalliques greffés sur un support organique ou minéral. Par incorporé, on entend que le métal est un élément du support ou que l'on travaille avec des complexes stériquement piégés dans des structures poreuses dans les conditions de l'oxydation.

Dans un mode de réalisation préféré de l'invention, le catalyseur homogène ou hétérogène est constitué de sels ou de complexes de métaux des groupes IVb (groupe du Ti), Vb (groupe du V), VIIb(groupe du Cr), VIIb (groupe du Mn), VIII (groupe du Fe ou Co ou Ni), Ib (groupe du Cu), et cérium, seuls ou en mélange. Les éléments préférés sont, en particulier, Co et/ou Mn et/ou Cr et/ou Zr, Hf, Ce et/ou Zr, Hf. La concentration en métal dans le milieu liquide d'oxydation varie entre 0,00001 et 5 % (% poids), de préférence entre 0,001 % et 2%.

L'invention concerne l'oxydation directe du cyclohexane en acide adipique, par un gaz contenant de l'oxygène, en milieu liquide et en présence d'un catalyseur. Le catalyseur comprend préférentiellement du cobalt.

La réaction d'oxydation est mise en oeuvre à une température comprise entre 50°C et 200°C, de préférence entre 70°C et 180°C. Elle peut être réalisée sous pression atmosphérique. Toutefois, elle est généralement mise en oeuvre sous pression pour maintenir les composants du milieu réactionnel sous forme liquide. La pression peut être comprise entre 10 KPa (0,1 bar) et 20000 KPa (200 bars), de préférence entre 100Kpa (1 bar) et 10000 Kpa (100 bar).

L'oxygène utilisé peut être sous forme pure ou en mélange avec un gaz inerte tel que l'azote ou l'hélium. On peut également utiliser de l'air plus ou moins enrichi en oxygène. La quantité d'oxygène alimentée dans le milieu est avantageusement comprise entre 1 et 1000 moles par mole de composés à oxyder.

Le procédé d'oxydation peut être réalisé de manière continue ou selon un procédé discontinu. Avantageusement, le milieu réactionnel liquide sorti du réacteur est traité selon des procédés connus permettant d'une part de séparer et récupérer l'acide produit et d'autre part recycler les composés organiques non oxydés ou partiellement oxydés comme le cyclohexane, le cyclohexanol et/ou le cyclohexanone, le catalyseur et le composé acide lipophile.

La quantité de catalyseur, exprimée en pourcentage pondéral de cobalt par rapport au mélange réactionnel, se situe généralement entre 0,00001 % et 5 % et de préférence entre 0,001 % et 2 %, sans que ces valeurs soient critiques. Il s'agit cependant d'avoir une activité suffisante tout en n'utilisant pas des quantités trop importantes d'un catalyseur qu'il faut ensuite séparer du mélange réactionnel final et recycler.

Le catalyseur, outre le cobalt, peut également comporter d'autres composés à base de métaux choisis dans le groupe comprenant le manganèse, le cuivre, le cérium, le vanadium, le chrome, le zirconium, l'hafnium ou une association de certains de ces éléments .

Il est avantageux de mettre en oeuvre également un composé initiateur de la réaction d'oxydation, tel que par exemple une cétone ou un aldéhyde, La cyclohexanone qui est un intermédiaire réactionnel dans le cas de l'oxydation du cyclohexane, est tout particulièrement indiquée. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation à une température inférieure à 120°C. Il peut être introduit dès le début de la réaction.

L'oxydation peut également être mise en oeuvre en présence d'eau introduite dès le stade initial du procédé.

Comme indiqué ci-dessus, le mélange réactionnel issu de l'oxydation est soumis à différentes opérations de séparation de certains de ses constituants pour, par exemple" permettre leur recyclage au niveau de l'oxydation et la récupération de l'acide adipique

Selon une première variante du procédé, on peut soumettre tout d'abord le mélange réactionnel brut à un refroidissement à une température de 16°C à 30°C par exemple, ce qui occasionne la cristallisation d'au moins une partie de l'acide formé. On obtient ainsi un milieu comprenant une phase solide constituée essentiellement d'acide, au moins une phase liquide organique contenant essentiellement le cyclohexane n'ayant pas réagi, éventuellement le composé acide lipophile et les intermédiaires d'oxydation, (ou plusieurs phases organiques si le composé acide lipophile et l'hydrocarbure ne sont pas totalement miscibles à basse température) et une phase liquide aqueuse contenant essentiellement des sous produits acides de l'oxydation et l'eau formée. Le catalyseur peut se trouver dans une des phases organiques s'il est soluble dans ladite phase, ou dans la phase aqueuse inférieure.

Après filtration ou centrifugation du solide, on procède s'il y a lieu à la séparation par décantation des phases liquides organiques et aqueuse constituant le filtrat ou le centrifugat : la ou les phases organiques peuvent être recyclées dans une nouvelle réaction d'oxydation.

Il peut être avantageux de procéder, préalablement à l'opération de cristallisation de l'acide, à une concentration du mélange réactionnel.

Selon une deuxième variante du procédé, on peut soutirer à chaud le mélange réactionnel brut final, par exemple à une température pouvant atteindre 75°C. Le mélange réactionnel décante alors en au moins deux phases liquides une ou plusieurs phases organiques contenant essentiellement cyclohexane n'ayant pas réagi, le composé acide lipophile les intermédiaires d'oxydation et une phase liquide aqueuse contenant essentiellement l'acide adipique et l'eau formée. Selon la solubilité et la nature du catalyseur celui-ci peut être présent dans la ou les phases organiques, récupéré par séparation solide/liquide avant précipitation ou cristallisation de l'acide formé dans le cas d'une catalyse hétérogène ou s'il est soluble dans la phase aqueuse, extrait par extraction liquide/liquide, sur résine ou électrodialyse.

Comme dans la première variante, on procède à la séparation par décantation des phases liquides : la ou les phases organiques peuvent être recyclées dans une nouvelle réaction d'oxydation.

Dans ces modes de réalisation, le composé acide lipophile utilisé conformément à l'invention est généralement contenu ou forme un élément essentiel de la ou des phases organiques. En conséquence, après séparation de l'acide adipique éventuellement de la phase liquide contenant l'eau formée, les sous-produits d'oxydation et le catalyseur, le composé acide lipophile est recyclé dans l'étape d'oxydation avec l'hydrocarbure n'ayant pas été oxydé et les intermédiaires d'oxydation.

Par ailleurs, si le composé acide lipophile est solide dans une phase de traitement du milieu réactionnel, il sera avantageusement séparé et récupéré par mise en oeuvre des procédés de séparation solide/liquide soit avant traitement du milieu réactionnel pour récupérer l'acide adipique, soit avec l'acide. Dans ce dernier cas, l'acide pourra être récupéré par extraction à l'eau.

Dans ces exemples de mode de réalisation de l'invention, de l'eau peut être ajouté au milieu réactionnel pour obtenir une meilleure dissolution des sous produits acides de l'oxydation et une meilleure récupération de l'acide adipique.

La récupération de l'acide est généralement réalisée par précipitation lors du refroidissement du milieu réactionnel. L'acide ainsi récupéré peut être purifié selon des techniques habituelles et décrites dans de nombreux brevets. On peut citer, à titre d'exemple, les brevets français n° 2749299 et 2749300.

Si la phase liquide non organique ou aqueuse contient le catalyseur celui-ci est extrait soit avant la cristallisation de l'acide formé par précipitation ou extraction selon des procédés connus comme l'extraction liquide - liquide, l'électrodialyse, traitement sur résines échangeuses d'ions par exemple, soit après cristallisation de l'acide formé par des techniques d'extraction décrites ci-dessus ou analogues.

D'autres avantages, détails de l'invention apparaîtront plus clairement au vu des exemples donnés ci-dessous uniquement à titre indicatif et d'illustration.

### Exemple 1 : Préparation du 3,5-ditertiobutyl benzoate de cobalt (II)

2, 42 g (10,3 mmol) d'acide 3,5-tertiobutyl benzoïque sont dissous dans 60 ml de cyclohexane à température ambiante (solution incolore). En parallèle on prépare une solution aqueuse constituée de 10 ml d'eau et 0,42 g de soude (10,5 mmol) à laquelle on ajoute 1,28 g (5,13 mmol) d'acétate de cobalt tétrahydraté dissous dans 10 ml d'eau (solution rose).

La solution aqueuse est ajoutée à la solution cyclohexanique sous agitation, à température ambiante. Au bout de 3 h, la phase aqueuse est incolore et la phase cyclohexanique bleu.

Après séparation des deux phases par décantation et élimination du cyclohexane par concentration, on récupère 2,69 g de cristaux bleu de 3,5 ditertiobutyl benzoate de cobalt (M=525,5).

### Exemple 2 comparatif:

Dans un autoclave de 125 ml en titane, muni de moyens de chauffage par collier chauffant, d'une turbine et de moyens d'introduction de gaz et de régulation de pression, on charge :
- 40,68 g (484,3 mmol) dé cyclohexane
- 4,87 g(33 mmol) d'acide 2-éthylhexanoique
- 0,4811 g (4,91 mmol) de cyclohexanone
- 0,42 g (0,80 mmol de Co) de 3,5 ditertiobutyl benzoate de cobalt de l'exemple 1.

Après fermeture du réacteur, on agite à 1000 tours par minute, on crée une pression d'air (100 bar à 20°C) et on chauffe. La température atteint 105°C dans la masse en 10 min et on maintient cette température pendant encore 3 heures.

Après refroidissement et dépressurisation, le mélange réactionnel comprend une phase comprenant le cyclohexane et un précipité.

Après séparation de la phase cyclohexanique, le précipité est dissous dans de l'acide acétique. La phase cyclohexanique et la solution acétique sont analysées par chromatographie en phase gazeuse, les résultats sont rassemblés dans le tableau I ci-dessous.
Le taux de transformation (TT) du cyclohexane est de : 2,0%

**TABLEAU I**

| Produits | Phase cyclohexanique mmol | Solution acétique Mmol | Total Mmol | ST % |
|---|---|---|---|---|
| Cyclohexanone* | 6,13 | 0,21 | 1,42* | 14,3 |
| Cyclohexanol | 5,45 | 0,19 | 5,64 | 57,0 |
| Butyrolactone | 0,08 | | 0,08 | <1 |
| Valérolactone | 0,05 | | 0,05 | <1 |
| Acide succinique | Traces | Traces | Traces | <1 |
| Acide glutarique | 0,17 | 0,24 | 0,41 | 4,1 |
| Acide adipique | 0,38 | 1,92 | 2,30 | 23,2 |

| | | | | |
|---|---|---|---|---|
| * cyclohexanone dosée - cyclohexanone initiale = cyclohexanone formée, ST% = sélectivité en composé indiqué à la 1^{ère} colonne par rapport au cyclohexane transformé. | | | | |

### Exemple :3

L'exemple 2 est répété mais en utilisant les produits de départ suivants :
- 40,34 g (480,37 mmol) de cyclohexane,
- 0,4688 g (478 mmol) de cyclohexanone,
- 2,67 g (11,4 mmol) d'acide 3,5-ditertiobutyl benzoique,
- 0,1078 g (0,205 mmol de Co) de 3,5 ditertiobutyl benzoate de cobalt;

Le taux de transformation (TT) du cyclohexane est de 1,26%

Les résultats de l'analyse du milieu réactionnel sont donnés dans le tableau V ci-dessous.

**TABLEAU V**

| Produits | Total mmol | ST % |
|---|---|---|
| Cyclohexanone* | 1,4 | 23,2 |
| Cyclohexanol | 3,76 | 62,4 |
| Butyrolactone | | |
| Valérolactone | | |
| Acide succinique | | |
| Acide glutarique | 0,11 | 1,8 |
| Acide adipique | 0,76 | 12,6 |

| | | |
|---|---|---|
| * cyclohexanone dosée - cyclohexanone initiale = cyclohexanone formée, | | |

## Revendications

1. Procédé d'oxydation de cyclohexane en milieu liquide par un agent d'oxydation comprenant de l'oxygène moléculaire en acide adipique **caractérisé en ce qu'**un des constituants du milieu liquide est un composé organique acide à caractère lipophile, choisi dans le groupe comprenant, les acides 2,5-ditertiobutyl benzoïque, 4-tertiobutylbenzoique, 4-octylbenzoïque, l'hydrogénoorthophtalate de tertiobutyle, les acides naphténiques ou anthracéniques substitués par des groupements alkyles, de préférence de type tertiobutyle, les dérivés substitués des acides phtaliques.

2. Procédé selon la revendication 1, **caractérisé en ce que** l'hydrocarbure à oxyder est au moins partiellement miscible avec le composé acide lipophile, aux conditions de mise en oeuvre de la réaction d'oxydation.

3. Procédé selon l'une des revendications précédentes, **caractérisé en ce que** le pourcentage pondéral de composés acides lipophiles dans le milieu liquide est compris entre 1 et 99 % en poids par rapport au poids total du milieu liquide.

4. Procédé selon la revendication 3, **caractérisé en ce que** le pourcentage pondéral précité est compris entre 10 et 80 % en poids.

5. Procédé selon l'une des revendications précédentes, **caractérisé en ce que** l'oxydation est réalisée en présence d'un catalyseur.

6. Procédé selon la revendication 5, **caractérisé en ce que** le catalyseur est soluble dans le milieu liquide aux conditions de mise en oeuvre de la réaction d'oxydation.

7. Procédé selon la revendication 5, **caractérisé en ce que** le catalyseur est insoluble dans le milieu liquide aux conditions de mise en oeuvre de la réaction d'oxydation.

8. Procédé selon la revendication 7, **caractérisé en ce que** le catalyseur est un catalyseur supporté comprenant un support minéral ou polymérique.

9. Procédé selon l'une des revendications précédentes, **caractérisé en ce que** l'oxydation est réalisée en présence d'un composé choisi dans la famille comprenant les nitriles, les composés hydroxyimides, les composés halogénés.

10. Procédé selon la revendication 9, **caractérisé en ce que** le composé précité est un composé nitrile choisi dans le groupe comprenant l'acétonitrile, le benzonitrile.

11. Procédé selon la revendication 9, **caractérisé en ce que** le composé précité est un composé hydroxyimide choisi dans le groupe comprenant la N-hydroxysuccinimide et la N-hydroxyphtalimide.

12. Procédé selon la revendication 9, **caractérisé en ce que** le composé précité est un composé halogéné choisi dans le groupe comprenant les hydrocarbures aliphatiques fluorés ou perfluorés cycliques ou acycliques, les hydrocarbures fluorés aromatiques tels le perfluorotoluène, perfluorométhylcyclohexane, perfluorohexane, perfluoroheptane, perfluorooctane, perfluorononane, perfluorodécaline, perfluorométhyldécaline, α,α,α-trifluorotoluène, 1,3-bis(méthyl trifluoro)benzène, les esters perfluorés ou fluorés tels que perfluorooctanoates d'alkyle, perfluoronanoates d'alkyle, les cétones fluorés ou perfluorés telles que acétone perfluorée, les alcools fluorés ou perfluorés tels que hexanol, octanol, nonanol, décanol perfluorés, t-butanol perfluoré, isopropanol perfluoré, hexafluoro-1,1,1,3,3,3-propanol-2, les nitriles fluorés ou perfluorés tels que acétonitrile perfluoré, les acides fluorés ou perfluorés tels que acides trifluorométhyl benzoïque, acide pentafluorobenzoique, acide hexanoique, heptanoique, octanoique, nonanoique perfluorés, acide adipique perfluoré, les halogénures fluorés ou perfluorés tels que iodo octane perfluoré, bromooctane perfluoré, les amines fluorées ou perfluorées tels que tripropylamine perfluorée, tributylamine perfluorée, tripentylamine perfluorée.

13. Procédé selon l'une des revendications précédentes, **caractérisé en ce que** le milieu liquide, après oxydation, est décanté, en au moins une phase organique formées par l'hydrocarbure non oxydé, le composé acide lipophile, lesdites phases organiques étant recyclées dans une nouvelle oxydation, l'acide produit étant extrait de la phase aqueuse.

14. Procédé selon la revendication 13, **caractérisé en ce que** l'acide est extrait de la phase aqueuse par cristallisation.

15. Procédé selon l'une des revendications 7, 13 ou 14, **caractérisé en ce que** le catalyseur est recyclé avec la ou les phases organiques.

16. Procédé selon l'une des revendications, 8 et 13 ou 14, **caractérisé en ce que** le catalyseur est séparé du milieu liquide par décantation ou séparation solide/liquide.

17. Procédé selon l'une des revendications 13 ou 14, **caractérisé en ce que** le catalyseur soluble dans la phase aqueuse est extrait par extraction liquide/liquide, séparation sur résines, ou par électrodialyse.

18. Procédé selon l'une des revendications précédentes, **caractérisé en ce que** le catalyseur comprend du cobalt comme élément catalytiquement actif.

## Patentansprüche

1. Verfahren zur Oxidation von Cyclohexan in flüssigem Medium durch ein Oxidationsmittel, das molekularen Sauerstoff umfasst, zu Adipinsäure, **dadurch gekennzeichnet, dass** einer der Bestandteile des flüssigen Mediums eine saure organische Verbindung mit lipophilem Charakter ist, die aus der Gruppe ausgewählt ist, die 2,5-Di-tert-butylbenzoesäure, 4-tert-Butylbenzoesäure, 4-Octylbenzoesäure, tert-Butyl-hydrogen-ortho-phthalat, die Naphthen- oder Anthracensäuren, die mit Alkylgruppen, vorzugsweise vom Typ tert-Butyl substituiert sind, die substituierten Derivate der Phthalsäuren umfasst.

2. Verfahren gemäß Anspruch 1, **dadurch gekennzeichnet, dass** der Kohlenwasserstoff, der oxidiert werden soll, wenigstens teilweise mit der lipophilen sauren Verbindung unter den Durchführungsbedingungen der Oxidationsreaktion mischbar ist.

3. Verfahren gemäß einem der vorhergehenden Ansprüche, **dadurch gekennzeichnet, dass** der Gewichtsprozentsatz von lipophilen sauren Verbindungen in dem flüssigen Medium, bezogen auf das Gesamtgewicht des flüssigen Mediums, zwischen 1 und 99 Gew.-% liegt.

4. Verfahren gemäß Anspruch 3, **dadurch gekennzeichnet, dass** der oben angeführte Gewichtsprozentsatz zwischen 10 und 80 Gew.-% liegt.

5. Verfahren gemäß einem der vorhergehenden Ansprüche, **dadurch gekennzeichnet, dass** die Oxidation in Gegenwart eines Katalysators durchgeführt wird.

6. Verfahren gemäß Anspruch 5, **dadurch gekennzeichnet, dass** der Katalysator in dem flüssigen Medium unter den Durchführungsbedingungen der Oxidationsreaktion löslich ist.

7. Verfahren gemäß Anspruch 5, **dadurch gekennzeichnet, dass** der Katalysator in dem flüssigen Medium unter den Durchführungsbedingungen der Oxidationsreaktion unlöslich ist.

8. Verfahren gemäß Anspruch 7, **dadurch gekennzeichnet, dass** der Katalysator ein trägergebundener Katalysator ist, der einen anorganischen oder polymeren Träger umfasst.

9. Verfahren gemäß einem der vorhergehenden Ansprüche, **dadurch gekennzeichnet, dass** die Oxidation in Gegenwart einer Verbindung durchgeführt wird, die aus der Familie ausgewählt ist, die die Nitrile, die Hydroxyimidverbindungen, die Halogenverbindungen umfasst.

10. Verfahren gemäß Anspruch 9, **dadurch gekennzeichnet, dass** die oben angeführte Verbindung eine Nitrilverbindung ist, die aus der Gruppe ausgewählt ist, die Acetonitril, Benzonitril umfasst.

11. Verfahren gemäß Anspruch 9, **dadurch gekennzeichnet, dass** die oben angeführte Verbindung eine Hydroxyimidverbindung ist, die aus der Gruppe ausgewählt ist, die N-Hydroxysuccinimid und N-Hydroxyphthalimid umfasst.

12. Verfahren gemäß Anspruch 9, **dadurch gekennzeichnet, dass** die oben angeführte Verbindung eine Halogenverbindung ist, die aus der Gruppe ausgewählt ist, die die cyclischen oder acyclischen fluorierten oder perfluorierten aliphatischen Kohlenwasserstoffe, die aromatischen fluorierten Kohlenwasserstoffe, wie Perfluortoluen, Perfluormethylcyclohexan, Perfluorhexan, Perfluorheptan, Pertluoroctan, Perfluomonan, Perfluordecalin, Perfluormethyldecalin, α,α,α-Trifluortoluen, 1,3-Bis(trifluormethyl)benzen, die perfluorierten oder fluorierten Ester, wie Perfluoroctansäurealkylester, Perfluornonansäure-atkytester, die fluorierten oder perfluorierten Ketone, wie perfluoriertes Aceton, die fluorierten oder perfluorierten Alkohole, wie perfluoriertes Hexanol, Octanol, Nonanol, Decanol, perfluoriertes t-Butanol, perfluoriertes Isopropanol, 1,1,1,3,3,3-Hexafluorpropan-2-ol, die fluorierten oder perfluorierten Nitrile, wie perfluoriertes Acetonitril, die fluorierten oder perfluorierten Säuren, wie Trifluormethylbenzoesäure, Pentafluorbenzoesäure, perfluorierte Hexan-, Heptan-, Octan-, Nonansäure, perfluorierte Adipinsäure, die fluorierten oder perfluorierten Halogenide, wie perfluoriertes lodoctan, perfluoriertes Bromoctan, die fluorierten oder perfluorierten Amine, wie perfluoriertes Tripropylamin, perfluoriertes Tributylamin, perfluoriertes Tripentylamin, umfasst.

13. Verfahren gemäß einem der vorhergehenden Ansprüche, **dadurch gekennzeichnet, dass** das flüssige Medium nach der Oxidation in wenigstens eine organische Phase dekantiert wird, die von dem nicht oxidierten Kohlenwasserstoff, der lipophilen sauren Verbindung gebildet wird, wobei besagte organische Phasen in eine neue Oxidation rückgeführt werden, die erzeugte Säure aus der wässrigen Phase extrahiert wird.

14. Verfahren gemäß Anspruch 13, **dadurch gekennzeichnet, dass** die Säure aus der wässrigen Phase durch Kristallisieren extrahiert wird.

15. Verfahren gemäß einem der Ansprüche 7, 13 oder 14, **dadurch gekennzeichnet, dass** der Katalysator mit der oder den organischen Phasen rückgeführt wird.

16. Verfahren gemäß einem der Ansprüche 8, 13 oder 14, **dadurch gekennzeichnet, dass** der Katalysator von dem flüssigen Medium durch Dekantieren oder Fest/Flüssig-Trennung abgetrennt wird.

17. Verfahren gemäß einem der Ansprüche 13 oder 14, **dadurch gekennzeichnet, dass** der in der wässrigen Phase lösliche Katalysator durch Flüssig/Flüssig-Extraktion, Trennung über Harzen oder durch Elektrodialyse extrahiert wird.

18. Verfahren gemäß einem der vorhergehenden Ansprüche, **dadurch gekennzeichnet, dass** der Katalysator Kobalt als katalytisch wirksames Element umfasst.

## Claims

1. Process for oxidizing cyclohexane to adipic acid in a liquid medium by an oxidizing agent comprising molecular oxygen, **characterized in that** one of the constituents of the liquid medium is an acidic organic compound which is of lipophilic nature, chosen from the group comprising 2,5-di-tert-butylbenzoic acid, 4-tert-butylbenzoic acid, 4-octylbenzoic acid, tert-butyl hydrogen ortho-phthalate, naphthenic or anthracenic acids substituted with alkyl groups, preferably of tert-butyl type, and substituted phthalic acid derivatives.

2. Process according to Claim 1, **characterized in that** the hydrocarbon to be oxidized is at least partially miscible with the lipophilic acidic compound, under the conditions in which the oxidation reaction is carried out.

3. Process according to one of the preceding claims, **characterized in that** the weight percentage of lipophilic acidic compounds in the liquid medium is between 1% and 99% by weight relative to the total weight of the liquid medium.

4. Process according to Claim 3, **characterized in that** the abovementioned weight percentage is between 10% and 80% by weight.

5. Process according to one of the preceding claims, **characterized in that** the oxidation is carried out in the presence of a catalyst.

6. Process according to Claim 5, **characterized in that** the catalyst is soluble in the liquid medium under the conditions in which the oxidation reaction is carried out.

7. Process according to Claim 5, **characterized in that** the catalyst is insoluble in the liquid medium under the conditions in which the oxidation reaction is carried out.

8. Process according to Claim 7, **characterized in that** the catalyst is a supported catalyst comprising a mineral or polymeric support.

9. Process according to one of the preceding claims, **characterized in that** the oxidation is carried out in the presence of a compound chosen from the family comprising nitriles, hydroxyimide compounds and halogenated compounds.

10. Process according to Claim 9, **characterized in that** the abovementioned compound is a nitrile compound chosen from the group comprising acetonitrile and benzonitrile.

11. Process according to Claim 9, **characterized in that** the abovementioned compound is a hydroxyimide compound chosen from the group comprising N-hydroxysuccinimide and N-hydroxyphthalimide.

12. Process according to Claim 9, **characterized in that** the abovementioned compound is a halogenated compound chosen from the group comprising cyclic or acyclic fluoroaliphatic or perfluoroaliphatic hydrocarbons and fluoroaromatic hydrocarbons, such as perfluorotoluene, perfluoromethylcyclohexane, perfluorohexane, perfluoroheptane, perfluorooctane, perfluorononane, perfluorodecalin, perfluoromethyldecalin, α,α,α-trifluorotoluene or 1,3-bis(trifluoromethyl)benzene; fluoro or perfluoro esters such as alkyl perfluorooctanoates and alkyl perfluorononanoates; fluoro or perfluoro ketones such as perfluoroacetone, fluoroalcohols or perfluoroalcohols such as perfluorohexanol perfluorooctanol, perfluorononanol, perfluorodecanol, perfluoro-t-butanol, perfluoroisopropanol or 1,1,1,3,3,3-hexafluoro-2-propanol, fluoronitriles or perfluoronitriles such as perfluoroacetonitrile, fluoro acids or perfluoro acids such as trifluoromethylbenzoic acid, pentafluorobenzoic acid, perfluorohexanoic acid, perfluoroheptanoic acid, perfluorooctanoic acid, perfluorononanoic acid or perfluoroadipic acid, fluoro or perfluoro halides such as perfluoroiodooctane and perfluorobromooctane, fluoroamines or perfluoroamines such as perfluorotripropylamine, perfluorotributylamine or perfluorotripentylamine.

13. Process according to one of the preceding claims, **characterized in that**, after oxidation, the phases of the liquid medium are separated by settling into at least one organic phase formed by the unoxidized hydrocarbon, the lipophilic acidic compound, the said organic phases being recycled into a further oxidation and the acid produced being extracted from the aqueous phase.

14. Process according to Claim 13, **characterized in that** the acid is extracted from the aqueous phase by crystallization.

15. Process according to one of Claims 7, 13 and 14, **characterized in that** the catalyst is recycled with the organic phase(s).

16. Process according to one of Claims 8, 13 and 14, **characterized in that** the catalyst is separated from the liquid medium by separation of the phases by settling or by solid/liquid separation.

17. Process according to either of Claims 13 and 14, **characterized in that** the catalyst which is soluble in the aqueous phase is extracted by liquid/liquid extraction, separation on resins or by electrodialysis.

18. Process according to one of the preceding claims, **characterized in that** the catalyst comprises cobalt as catalytically active element.
